# EUROPEAN PATENT SPECIFICATION

(11) **EP 0 567 443 B1**
(45) Date of publication and mention of the grant of the patent: **31.07.1996**
(21) Application number: 93870055.6
(22) Date of filing: 26.03.1993
(51) Int. Cl.: C07C 5/27, C07C 9/12, C07C 4/10

(54) **Hydrocarbon conversion process**
Verfahren zur Umwandlung von Kohlenwasserstoffen
Procédé pour la conversion d'hydrocarbures

(30) Priority: 26.03.1992 US 857857
(43) Date of publication of application: 27.10.1993
(73) Proprietor: FINA TECHNOLOGY, INC., Dallas, Texas 75206 (US)
(72) Inventor: Shamshoum, Edwar, Houston, Texas 77062 (US); Ghosh, Ashim, Houston, Texas 77062 (US); Schuler, Thomas, Galena Park, Texas 77547 (US)
(74) Representative: Leyder, Francis

(56) References cited:
- FR-A- 2 150 707
- FR-A- 2 183 520
- FR-A- 2 233 097
- US-A- 4 962 250

## Description

### TECHNICAL FIELD OF THE INVENTION

This invention relates the use of a platinum metal-loaded X zeolite catalyst into a process for converting normal paraffins to their higher octane isomer counterparts and/or to isobutane.

### BACKGROUND OF THE INVENTION

The isomerization of normal paraffins has long been an important refinery process. The resulting isomerization products have significantly higher octane values (RON) than their normal paraffin counterparts. For example, isomerization of normal heptane to methylhexanes and dimethylpentanes increases the RON value by approximately 50 and 90, respectively. Similarly, isomerization of normal octane to methylheptanes and dimethylhexanes results in an increased RON value of about 35 and 85, respectively. These higher octane isomerization products can then be blended with gasoline to increase its octane value.

Various catalysts have been employed to catalyze such isomerization reactions, including Friedal-Crafts catalysts (e.g. aluminum chloride), noble-metal catalysts, and crystalline aluminosilicate zeolite catalysts, such as zeolite X, Y, and synthetic mordenite. Further, zeolite-based bi-functional catalysts have also been used in hydroisomerization processes. The process operates at a relatively low hydrogen pressure owing to the high activity of the zeolite catalyst. Incorporation of a metal into the zeolite catalyst functions to dehydrogenate the normal paraffins to olefins. The olefins are then converted to carbocations on the acidic zeolite and desorbed as isoolefins, which are then hydrogenated again on the metal to give the corresponding isoparaffins.

French patent 2183520 discloses a process for the preparation of isopentane through hydro-isomerization of piperylene or a mixture of piperylene and n-pentane in the presence of a Y zeolite granulated with a binder. The purpose of this invention is to increase the ressources of raw material for the preparation of isopentane.

Another important refinery process is the conversion of normal paraffins to more desirable products such as isobutane.
Isobutane is a valuable product that can be used to produce alkylates, or can be selectively dehydrogenated to isobutylene for use in MTBE (methyl tert-butyl ether) processes. Demand for high octane oxygenates such as MTBE, as well as for isoparaffins and alkylates, will likely increase in view of federal regulations requiring a reduction in the vapor pressure and aromatics content of gasoline.

It can be appreciated that, at any given time, the prevailing economic conditions will have a considerable impact on determining whether it is more desirable to isomerize a feedstock of normal paraffins to their higher octane isomer counterparts, or to convert them to isobutane. What would be advantageous, therefore, is a catalyst and process that could be used for the isomerization of normal paraffins and/or the conversion of normal paraffins to isobutane.

### SUMMARY OF THE INVENTION

In accordance with the present invention, there is provided a novel process for converting normal paraffins to more valuable products, such as their higher octane isomer counterparts and/or isobutane. The present invention may be utilized to obtain high yields of liquid isomerization products having significantly higher octane (RON) values than the normal paraffin feed, while at the same time producing substantial amounts of isobutane. The process comprises contacting the normal paraffin feed with a hydrocarbon conversion catalyst under conversion conditions whereby the corresponding isoparaffins and/or isobutane are formed. The hydrocarbon conversion catalyst is a platinum metal-loaded X zeolite which has been fully ion-exchanged with lanthanum ions.

A platinum/lanthanum X (Pt/LaX) catalyst suitable for use in the present process is preferably prepared by first fully ion-exchanging a "parent" or as synthesized X zeolite with lanthanum ions and then ion-exchanging this lanthanum containing form of the zeolite with platinum ions. The platinum and lanthanum ion-exchanged zeolite is extruded with a binder, such as alumina or silica, and is then calcined. Alternatively, platinum can be incorporated into the catalyst by wet impregnation of the lanthanum X zeolite after it has been extruded with a binder.

As noted above, the present invention relates to a process for conveying normal paraffins to their corresponding isoparaffins and/or to isobutane. It is believed that isobutane formation occurs primarily as a result of hydrocracking of the isomerization products, but the isomerizatlon of normal butane resulting from hydrocracking of the normal paraffin feed may also account for some isobutane production. The isobutane can then be selectively dehydrogenated to isobutylene and subsequently utilized to produce MTBE (methyl tertiary-butyl ether).

In another embodiment of the present invention, isobutane yields approaching 50% can be achieved by operating at higher conversion reaction temperatures. At such higher reaction temperatures, however, little or no liquid isomerization product can be recovered.

### BRIEF DESCRIPTION OF THE DRAWINGS

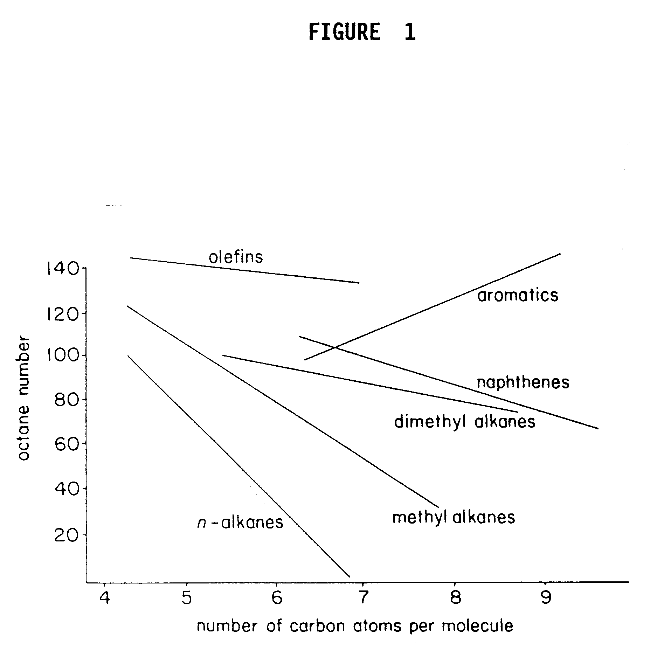

FIGURE 1 is a graphical representation showing the octane values of hydrocarbons as a function of the number of carbon atoms per molecule.

### DETAILED DESCRIPTION OF THE INVENTION

The present invention relates to a process for converting normal paraffins to their corresponding isoparaffins and/or to isobutane. FIGURE 1 graphically illustrates how such isomerization of normal paraffins increases the octane value of such hydrocarbons, The present process employs a hydrocarbon conversion catalyst comprising a platinum/lanthanum X (Pt/LaX) zeolite, having about 0.2 to 2.0 weight percent platinum and 5 to 12 weight percent lanthanum, with 0.4 to 1.2 weight percent platinum and 8 to 10 weight percent lanthanum being preferred.

In one embodiment a hydrocarbon feedstock containing normal paraffins is supplied to a reaction zone where it is brought into contact with a Pt/LaX zeolite conversion catatyst under conversion reaction conditions sufficient to cause isomerization of at least a portion of the normal paraffins, and thereby produce a liquid isomerization product having a RON value greater than that of the paraffin feed. Such an increase in the octane value of normal paraffins is obtained in accordance with the present invention without producing substantial amounts (i.e. less than 10 wt.%) of aromatic compounds.

Hydrogen is also supplied to the reaction zone, preferably as a co-feed with the hydrocarbon feedstock. Preferred hydrogen/hydrocarbon feed molar ratios are between about 1 and 10, with about 3 to 5 being more preferred. Conversion reaction conditions include a temperature of between about 190°C and 300°C, with about 220°C to 270°C being preferred and a pressure between about 689.7 and 3448.3 kPa (100 and 500 psig), with about 1379.3 kPa (200 psig) being preferred. Additionally, liquid hourly space velocities (LHSV) of between about 0.5 and 5.0 are utilized for the hydrocarbon feed with an LHSV of approximately 1 to 3 being most preferred.

The present invention has utility for conversion of hydrocarbon feedstocks containing normal C₅ to C₁₂ paraffins. By practicing the process of the present invention, liquid yields (C₅₊) of between 70 and 90 wt.% having RON values of between about 55 and 40 respectively, can be obtained. The specific liquid yield and RON value is dependent primarily upon the reaction temperature, the selection of which may be governed by economic conditions. In addition to converting the normal paraffins to their corresponding isomers, significant amounts of isobutane will also be produced. Even when operating at conversion conditions most suitable for isomerization, isobutane yields up to about 15 to 20 wt.% can be obtained. If higher yields of isobutane are desired, higher conversion temperatures (e.g. 250 - 320°C, with 280 to 300°C preferred) are employed allowing yields of isobutane in excess of 40 wt.% to be obtained. At such higher temperatures, however, little or no liquid isomerization product can be recovered.

A Pt/LaX catalyst suitable for use in the present invention can be prepared by modification of a "parent" crystalline X zeolite having a silica to alumina molar ratio (SiO₂/Al₂O₃) of between about 1 and 3. Basic procedures for the preparation of such a "parent" zeolite X are well known in the art and need not be detailed herein. In accordance with a preferred modification method, the parent X zeolite is first subjected to repeated lanthanum ion-exchanges to obtain the maximum loading of lanthanum ions in the zeolite. An aqueous solution of an inorganic lanthanum salt, preferably lanthanum nitrate, is employed as the ion-exchange medium. This lanthanum-containing form of the X zeolite powder is then subjected to platinum ion-exchange. Such platinum ion exchange is preferably accomplished by utilization of an aqueous platinum salt solution, such as tetraamineplatinum chloride.

After the platinum and lanthanum ions are incorporated into the X zeolite, it is then mixed with a binder, such as alumina sol, silica, gamma-alumina or other refractory oxides. This mixture is then pelletized by a suitable technique, such as extrusion, and the resulting pellets calcined at a maximum calcination temperature of 530°C. The resulting pt/LaX zeolite preferably contains about 0.2 to 2.0 weight percent platinum and 5 to 12 weight percent lanthanum prior to extrusion with the binder, with 0.4 to 1.2 weight percent platinum and 8 to 10 weight percent lanthanum being preferred.

An alternative method for incorporating platinum into the catalyst is via wet impregnation of the lanthanum containing X zeolite alter it has been extruded with a binder. Tetraamineplatinum chloride is preferably employed as the wet impregnation medium.

The following experimental work will serve to more fully describe the present invention. It is understood that these examples are not intended to limit the true scope of the invention, but rather are presented for illustrative purposes.

In experimental work carried out in accordance with the present invention, a Pt/LaX catalyst was employed for the conversion of a mixed feed containing approximately sixty percent normal heptane and forty percent normal octane to their corresponding isomers (iC₇ and iC₈) and/or to isobutane (iC₄).

### Preparation of Catalyst

A parent, or "as synthesized" X Zeolite was fully ion-exchanged with lanthanum ions in an aqueous solution of lanthanum nitrate. Three successive lanthanum ion-exchanges were employed to obtain a maximum lanthanum ion loading in the zeolite. The zeolite powder was then filtered, washed and dried. Platinum was ion-exchanged into the LaX zeolite powder utilizing an aqueous solution of tetraamineplatinum chloride. After filtration, washing, and drying, the Pt/LaX sample was extruded with twenty weight percent alumina as a binder and calcined at a maximum temperature of 530°C for two hours. The final catalyst contained 1.0 wt.% platinum and 9.98 wt.% lanthanum. The extruded catalyst had a SiO₂/Al₂O₃ molar ratio of 1.18.

### Conversion of Normal Paraffins

The Pt/LaX catalyst prepared above was utilized in the conversion of a mixed hydrocarbon feed containing approximately sixty percent normal heptane and forty percent normal octane to their corresponding isomers (iC₇ and iC₈) and/or to isobutane (iC₄). Approximately 35 ml of the catalyst was loaded into a micro-reactor and activated by heating under hydrogen flow (0.4 l/min.), at a rate of 75°C per hour for the first two hours, followed by 50°C per hour until 350°C was reached. The system was left at this temperature overnight to complete activation. The temperature was then dropped to 190°C at which time the feed containing sixty percent n-heptane and forty percent n-octane was introduced at a rate to give a LHSV of approximately 1.7. A reaction pressure of 1379.3 kPa (200 psig) was utilized. Hydrogen was used as a co-feed and was adjusted to give an H₂/hydrocarbon feed molar ratio of approximately 4.6. The reactor temperature was increased by 10°C increments until the hydrocarbon feed was converted completely into gaseous products.

The results obtained are listed below in TABLE I.

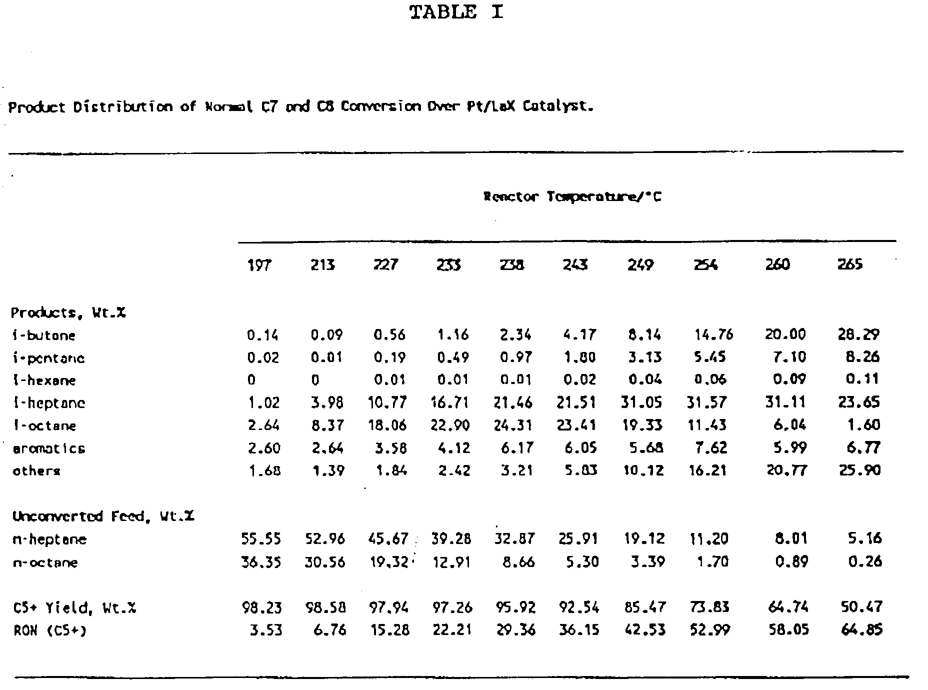

A review of TABLE I indicates that the present invention can be utilized to give 70 to 90 weight percent liquid yields (C₅₊) having RON values of approximately 55 and 40, respectively, at temperatures between 220°C and 250°C. In this temperature range, the isomerization product yields range between about 25 and 50 weight percent, and the iC₄ yield is 0.5 to 10 weight percent.

It can be seen that isobutane yields about 30 weight percent were obtained by operating at higher temperatures. At such high temperatures, the liquid yield begins dropping rapidly and primarily gaseous products are produced.

Having described specific embodiments of the present invention, it will be understood that modification thereof may be suggested to those skilled in the art, and it is intended to cover all such modifications as falls within the scope of the appended Claims.

## Claims

1. A process for the conversion of normal paraffins comprising passing a hydrocarbon feedstream containing the normal paraffins through a reaction zone under conversion conditions effective to cause conversion of at least a portion of the normal paraffins to an isomerized hydrocarbon product, wherein
(1) the reaction zone contains a platinum/lanthanum X (Pt/La X) zeolite catalyst and
(2) the conversion conditions comprise :
(a) Temperatures in the range of 190°C to 300°C;
(b) Pressures in the range of 689.7 kPa to 3448.3 kPa (100 psig to 500 psig); and
(c) Liquid hourly space velocities of the hydrocarbon feedstream between 0.5 and 5.0.

2. The process as recited in Claim 1 wherein the normal paraffins have between 5 and 12 carbon atoms per molecule.

3. The process as recited in Claim 2 wherein the normal paraffins have between 5 and 8 carbon atoms per molecule.

4. The process a recited in Claim 1 wherein hydrogen is supplied to the reaction zone as a co-feed with the hydrocarbon feedstock at a hydrogen/hydrocarbon feedstock molar ratio between 1 and 10.

5. The process as recited in Claim 1 wherein a liquid C₅₊ hydrocarbon product yield of at least 70 weight percent, based upon the normal paraffin feed, is obtained.

6. The process as recited in Claim 5 wherein the liquid C₅₊ hydrocarbon product has a research octane number of at least 50.

7. The process as recited in Claim 1 wherein a liquid C₅₊ hydrocarbon product yield of at least 90 weight percent, based upon the normal paraffin feed, is obtained.

8. The process as recited in Claim 7 wherein the liquid C₅₊ hydrocarbon product has a research octane number of at least 30.

9. The process as recited in Claim 1 wherein at least a portion of the normal paraffins are converted to isobutane.

10. The process as recited in Claim 5 wherein at least a portion of the normal paraffins are converted to isobutane and an isobutane yield of at least 10 weight percent , based upon the normal paraffin feed, is obtained.

11. The process as recited in Claim 1 wherein the catalyst contains 0.2 to 2.0 weight percent platinum and 5 to 12 weight percent lanthanum.

12. The process as recited in Claim 11 wherein the catalyst contains 0.4 to 1.2 weight percent platinum and 8 to 10 weight percent lanthanum.

13. A process for converting normal paraffins to isobutane comprising passing a hydrocarbon feedstream containing the normal paraffins through a reaction zone under conversion conditions effective to cause conversion of at least a portion of the normal paraffins to isobutane, wherein
(1) the reaction zone contains a platinum/lanthum X (Pt/la X) zeolite catalyst and
(2) the conversion conditions comprise :
(a) Temperatures in the range of 250°C to 320°C;
(b) Pressures in the range of 689.7 kPa to 3448.3 kPa (100 psig to 500 psig); and
(c) Liquid hourly space velocities of the hydrocarbon feed between 0.5 and 5.0.

14. The process as recited in Claim 13 wherein the normal paraffins have between 5 and 12 carbon atoms per molecule.

15. The process as recited in Claim 14 wherein the normal paraffins have between 5 and 8 carbon atoms per molecule.

16. The process as recited in Claim 13 wherein hydrogen is supplied to the reaction zone as a co-feed with the hydrocarbon feedstock at a hydrogen/hydrocarbon feed molar ratio between 1 and 10.

17. The process as recited in Claim 13 wherein the weight percent yield of isobutane based upon the normal paraffin feed is at least 25.

18. The process as recited in Claim 13 wherein the catalyst contains 0.2 to 2.0 weight percent platinum and 5 to 12 weight percent lanthanum.

19. The process as recited in Claim 18 wherein the catalyst contains 0.4 to 1.2 weight percent platinum and 8 to 10 weight percent lanthanum.

## Patentansprüche

1. Verfahren zur Umwandlung von Normalparaffinen durch Hindurchleiten eines Kohlenwasserstoffspeisestroms, der die Normalparaffine enthält, durch eine Reaktionszone unter Umwandlungsbedingungen, die den Effekt haben, die Umwandlung von wenigstens einem Teil der Normalparaffine in ein isomerisiertes Kohlenwasserstoffprodukt zu bewirken, wobei
(1) die Reaktionszone einen Platin/Lanthan X (Pt/La X) - Zeolith-Katalysator enthält, und
(2) die Umwandlungsbedingungen beinhalten:
(a) Temperaturen in dem Bereich von 190 °C bis 300 °C;
(b) Drücke in dem Bereich von 689,7 kPa bis 3448,3 kPa (100 psig bis 500 psig); und
(c) Flüssigkeitsstundenraumgeschwindigkeiten des Kohlenwasserstoffspeisestroms zwischen 0,5 und 5,0.

2. Verfahren nach Anspruch 1, wobei die Normalparaffine zwischen 5 und 12 Kohlenstoffatome pro Molekül haben.

3. Verfahren nach Anspruch 2, wobei die Normalparaffine zwischen 5 und 8 Kohlenstoffatome pro Molekül haben.

4. Verfahren nach Anspruch 1, wobei Wasserstoff der Reaktionszone als zusätzliche Speisung zusammen mit dem Kohlenwasserstoffspeisematerial mit einem Wasserstoff/Kohlenwasserstoff-Speisematerialmolverhältnis zwischen 1 und 10 zugeführt wird.

5. Verfahren nach Anspruch 1, wobei eine Ausbeute an flüssigem C₅₊-Kohlenwasserstoffprodukt von wenigstens 70 Gewichtsprozent, auf der Basis der Normalparaffinspeisung, erzielt wird.

6. Verfahren nach Anspruch 5, wobei das flüssige C₅₊-Kohlenwasserstoffprodukt eine Versuchsoktanzahl von wenigstens 50 hat.

7. Verfahren nach Anspruch 1, wobei eine Ausbeute an flüssigem C₅₊-Kohlenwasserstoffprodukt von wenigstens 90 Gewichtsprozent, auf der Basis der Normalparaffinspeisung, erzielt wird.

8. Verfahren nach Anspruch 7, wobei das flüssige C₅₊-Kohlenwasserstoffprodukt eine Versuchsoktanzahl von wenigstens 30 hat.

9. Verfahren nach Anspruch 1, wobei wenigstens ein Teil der Normalparaffine in Isobutan umgewandelt wird.

10. Verfahren nach Anspruch 5, wobei wenigstens ein Teil der Normalparaffine in Isobutan umgewandelt wird und eine Isobutanausbeute von wenigstens 10 Gewichtsprozent, auf der Basis der Normalparaffinspeisung, erzielt wird.

11. Verfahren nach Anspruch 1, wobei der Katalysator 0,2 bis 2,0 Gewichtsprozent Platin und 5 bis 12 Gewichtsprozent Lanthan enthält.

12. Verfahren nach Anspruch 11, wobei der Katalysator 0,4 bis 1,2 Gewichtsprozent Platin und 8 bis 10 Gewichtsprozent Lanthan enthält.

13. Verfahren zum Umwandeln von Normalparaffinen in Isobutan durch Hindurchleiten eines Kohlenwasserstoffspeisestroms, der die Normalparaffine enthält, durch eine Reaktionszone unter Umwandlungsbedingungen, die den Effekt haben, daß die Umwandlung von wenigstens einem Teil der Normalparaffine in Isobutan bewirkt wird, wobei
(1) die Reaktionszone einen Platin/Lanthan X (Pt/La X) - Zeolith-Katalysator enthält und
(2) die Umwandlungsbedingungen beinhalten:
(a) Temperaturen in dem Bereich von 250 °C bis 320 °C;
(b) Drücke in dem Bereich von 689,7 kPa bis 3448,3 kPa (100 psig bis 500 psig); und
(c) Flüssigkeitsstundenraumgeschwindigkeiten der Kohlenwasserstoffspeisung zwischen 0,5 und 5,0.

14. Verfahren nach Anspruch 13, wobei die Normalparaffine zwischen 5 und 12 Kohlenstoffatome pro Molekül haben.

15. Verfahren nach Anspruch 14, wobei die Normalparaffine zwischen 5 und 8 Kohlenstoffatome pro Molekül haben.

16. Verfahren nach Anspruch 13, wobei Wasserstoff der Reaktionszone als zusätzliche Speisung zusammen mit dem Kohlenwasserstoffspeisematerial mit einem Wasserstoff/Kohlenwasserstoff-Speisungsmolverhältnis zwischen 1 und 10 zugeführt wird.

17. Verfahren nach Anspruch 13, wobei die Ausbeute an Isobutan in Gewichtsprozent, auf der Basis der Normalparaffinspeisung, wenigstens 25 beträgt.

18. Verfahren nach Anspruch 13, wobei der Katalysator 0,2 bis 2,0 Gewichtsprozent Platin und 5 bis 12 Gewichtsprozent Lanthan enthält.

19. Verfahren nach Anspruch 18, wobei der Katalysator 0,4 bis 1,2 Gewichtsprozent Platin und 8 bis 10 Gewichtsprozent Lanthan enthält.

## Revendications

1. Un procédé de conversion des paraffines normales qui comprend le passage d'un flux d'alimentation hydrocarboné comprenant les paraffines normales au travers d'une zone de réaction sous des conditions de conversion effectives pour provoquer la conversion d'au moins une partie des paraffines normales en un produit hydrocarboné polymérisé, où
(1) la zone de réaction contient un catalyseur zéolite platine/lanthane X (Pt/La X) et
(2) les conditions de conversion comprennent :
(a) des températures comprises entre 190°C et 300°C;
(b) des pressions comprises entre 689.7 kPa et 3448.3 kPa (entre 100 psig et 500 psig); et
(c) des vitesses spatiales horaires du liquide du flux d'alimentation hydrocarboné comprises entre 0.5 et 5.0.

2. Le procédé tel qu'énuméré dans la revendication 1 où les paraffines normales comprennent entre 5 et 12 atomes de carbone par molécule.

3. Le procédé tel qu'énuméré dans la revendication 2 où les paraffines normales comprennent entre 5 et 8 atomes de carbone par molécule.

4. Le procédé tel qu'énuméré dans la revendication 1 où de l'hydrogène est apporté dans la zone de réaction en tant que co-alimentation avec le flux d'alimentation hydrocarboné dans un rapport molaire hydrogène/hydrocarbure au sein du flux d'alimentation compris entre 1 et 10.

5. Le procédé tel qu'énuméré dans la revendication 1 où un produit hydrocarboné C5+ liquide est obtenu avec un rendement d'au moins 70% en poids par rapport à l'alimentation en paraffine normale.

6. Le procédé tel qu'énuméré dans la revendication 5 où le produit hydrocarboné C5+ liquide possède un nombre d'octane de recherche d'au moins 50.

7. Le procédé tel qu'énuméré dans la revendication 1 où un produit hydrocarboné C5+ liquide est obtenu avec un rendement d'au moins 90% en poids par rapport à l'alimentation en paraffine normale.

8. Le procédé tel qu'énuméré dans la revendication 7 où le produit hydrocarboné C5+ liquide possède un nombre d'octane de recherche d'au moins 30.

9. Le procédé tel qu'énuméré dans la revendication 1 où au moins une partie des paraffines normales sont converties en isobutane.

10. Le procédé tel qu'énuméré dans la revendication 5 où au moins une partie des paraffines normales sont converties en isobutane et où un rendement en isobutane d'au moins 10 % en poids est obtenu par rapport à l'alimentation en paraffine normale.

11. Le procédé tel qu'énuméré dans la revendication 1 où le catalyseur contient entre 0.2 et 2.0 pour-cent en poids de platine et entre 5 et 12 pour-cent en poids de lanthane.

12. Le procédé tel qu'énuméré dans la revendication 11 où le catalyseur contient entre 0.4 et 1.2 pour-cent en poids de platine et entre 8 et 10 pour-cent en poids de lanthane.

13. Un procédé de conversion des paraffines normales en isobutane qui comprend le passage d'un flux d'alimentation hydrocarboné comprenant les paraffines normales au travers d'une zone de réaction sous des conditions de conversion effectives pour provoquer la conversion d'au moins une partie des paraffines normales en isobutane, où
(1) la zone de réaction contient un catalyseur zéolite platine/lanthane X (Pt/La X) et
(2) les conditions de conversion comprennent :
(a) des températures comprises entre 250°C et 320°C;
(b) des pressions comprises entre 689.7 kPa et 3448.3 kPa (entre 100 psig et 500 psig); et
(c) des vitesses spatiales horaires du liquide du flux d'alimentation hydrocarboné comprises entre 0.5 et 5.0.

14. Le procédé tel qu'énuméré dans la revendication 13 où les paraffines normales comprennent entre 5 et 12 atomes de carbone par molécule.

15. Le procédé tel qu'énuméré dans la revendication 14 où les paraffines normales comprennent entre 5 et 8 atomes de carbone par molécule.

16. Le procédé tel qu'énuméré dans la revendication 13 où de l'hydrogène est apporté dans la zone de réaction en tant que co-alimentation avec le flux d'alimentation hydrocarboné dans un rapport molaire hydrogène/hydrocarbure au sein du flux d'alimentation compris entre 1 et 10.

17. Le procédé tel qu'énuméré dans la revendication 13 où le pourcentage de rendement en poids en isobutane basé sur l'alimentation en paraffine normale est d'au moins 25.

18. Le procédé tel qu'énuméré dans la revendication 13 où le catalyseur contient entre 0.2 et 2.0 pour-cent en poids de platine et entre 5 et 12 pour-cent en poids de lanthane.

19. Le procédé tel qu'énuméré dans la revendication 18 où le catalyseur contient entre 0.4 et 1.2 pour-cent en poids de platine et entre 8 et 10 pour-cent en poids de lanthane.
